# EUROPÄISCHE PATENTANMELDUNG

(11) **EP 3 263 618 A1**
(43) Veröffentlichungstag der Anmeldung: **03.01.2018**
(21) Anmeldenummer: 16176308.1
(22) Anmeldetag: 27.06.2016
(51) Int. Cl.: C08G 18/73, C08G 18/75, C08G 18/24, C09D 175/12, C08G 18/32, C08G 18/38, C08G 18/80, C07F 7/18

(54) **ALKOXYSILAN-FUNKTIONALISIERTE ALLOPHANATE**

(71) Anmelder: Evonik Degussa GmbH, 45128 Essen (DE)
(72) Erfinder: Stache, Wiebke, 45699 Herten (DE); Unkelhäusser, Tobias, 48249 Dülmen (DE); Spyrou, Emmanouil, 46514 Schermbeck (DE); Lilienthal, Annegret, 46282 Dorsten (DE); Brückner, Iris, 46284 Dorsten (DE); Pfingsten, Jan, 44577 Castrop-Rauxel (DE)

(57) **Zusammenfassung**

Die vorliegende Erfindung betrifft Alkoxysilan-funktionalisierte Allophanate, Verfahren zur ihrer Herstellung, und ihre Verwendung.

## Beschreibung

Die vorliegende Erfindung betrifft Alkoxysilan-funktionalisierte Allophanate, ein Verfahren zur ihrer Herstellung, und ihre Verwendung.

Polyurethane haben sich seit vielen Jahrzehnten als hochwertige Bausteine für Lack-, Klebstoff-, Dichtstoff- und Kunststoffsysteme erwiesen. Dabei können zusätzliche Alkoxysilangruppen hier eine wichtige Rolle z. B. in Hinblick auf Netzwerkdichte, Chemikalienbeständigkeit und Kratzfestigkeit spielen, in erster Linie durch die Ausbildung von Siloxan- und Polysiloxanstrukturen.

Moleküle, die sowohl über Alkoxysilangruppen verfügen, als auch Isocyanatgruppen aufweisen, bieten die Möglichkeit, die als Reaktionsprodukte resultierende Funktionalitäten, Siloxane und Polyurethangruppen, durch eine Komponente einzufügen. Auch solche Substanzen sind schon lange in Gebrauch, z. B. in Form von Isocyanatoalkyltrialkoxysilanen.

Aus Isocyanatoalkyltrialkoxysilanen und Alkoholen hergestellte Alkoxysilan-terminierte Polyurethane sind ebenfalls bekannt und werden beispielsweise für die Herstellung hochvernetzter, harter Beschichtungsmitteln (z.B. WO 2013/189882) eingesetzt. Werden diese Alkoxysilan-terminierten Polyurethane jedoch als alleinige Bindemittel in Raumtemperaturhärtenden Systemen eingesetzt, so erhält man Beschichtungen mit nur mäßiger Härte.
Es gibt daher einen Bedarf für neuartige Bindemittel, die den Nachteil des Standes der Technik überwinden.

Allophanat-haltige Bindemittel sind lange bekannt. Auch Alkoxysilan-funktionalisierten Allophanate sind bekannt. Hier gilt es verschiedene Typen zu unterscheiden, die im Folgenden dargestellt werden, aber weder in der Struktur noch in der Anwendung der erfindungsgemäßen Alkoxysilan-funktionalisierten Allophanate entsprechen.

So werden die in WO2008/043722 beschriebenen Allophanate III (1) durch Umsetzung NCOterminierter allophanathaltige Polyurethane I (1) mit gegenüber Isocyanat reaktiven Alkoxysilanen II (1) (z.B. Aminoalkyltrialkoxysilan) erhalten. Die Allophanat-Gruppen befinden sich hier somit im Zentrum der Polyurethankette und die Alkoxysilanfunktion ist über die terminale Isocyanat-Gruppe im Rahmen einer Harnstoff-Funktion angebunden (Struktur III (1), Gleichung 1).

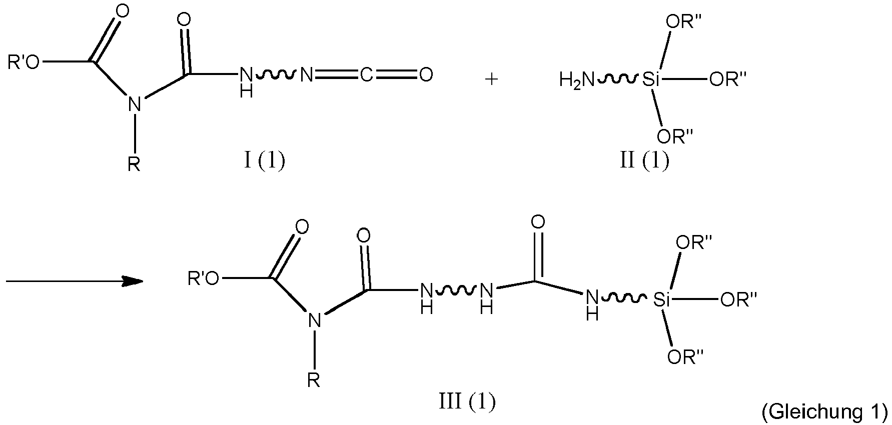

DE102005041953 beschreibt die Umsetzung eines Polyols I (2) mit einem mittleren Molekulargewicht von 3000 - 20000 g/Mol mit einem Überschuss von Isocyanatopropyltrimethoxysilan II (2), so dass es nach der Polyurethanbildung III (2) zu Bildung eines Allophanates IV (2) mit zwei Alkoxysilanfunktionen pro Allophanateinheit kommt.

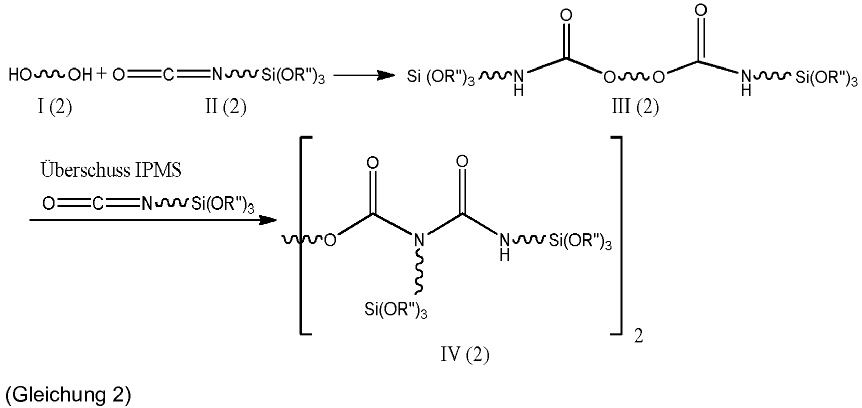

In DE102005041954 wird ein Polyurethan I (3) mit Isocyanatopropyltrimethoxysilan II (3) versetzt und so lange erhitzt bis sich Allophanatstrukturen ausbilden. In diesem Fall wird die Alkoxysilangruppe an dem terminalen Stickstoff der Allophanat-Gruppe III (3) angebaut (Gleichung 3).

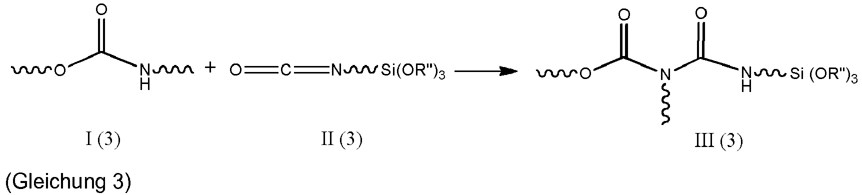

J. Kozakiewicz et al. publizierten in Progress in Organic Coatings 72 (2011) 120-130 die Umsetzung von Isocyanatopropyltrimethoxysilan I (4) mit Methanol zum entsprechenden Urethan II (4) und anschließend mit Hexamethylendiisocyanat-Trimer III (4). Bei dem hieraus resultierenden hochviskosen Allophanat IV (4) hängt die Alkoxysilanfunktion an dem tertiären, zentralen Amin der Allophanat-Gruppe (Gleichung 4).

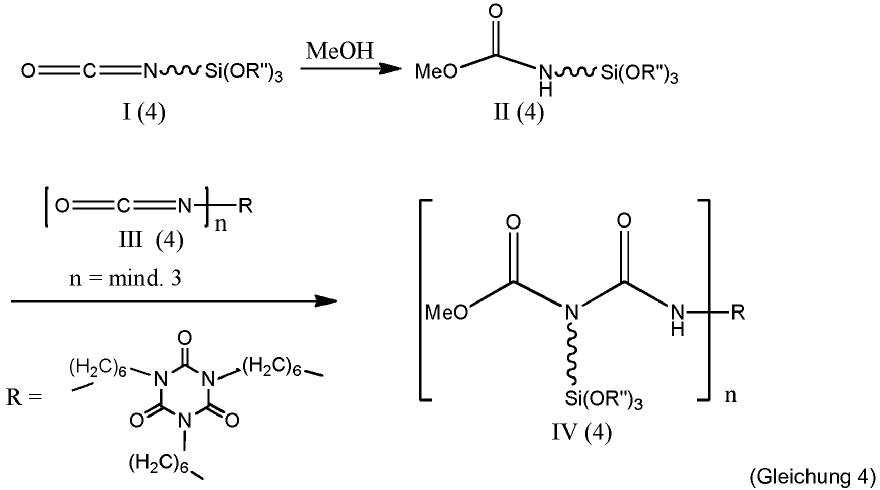

Die Allophanat-Funktion dient in der beschriebenen Anwendung als Blockierungsmittel für das Hexamethylendiisocyanat-Trimer, welches als Vernetzer für Hydroxy-funktionalisierte Polyesterpolyole eingesetzt wurde.

Auch heute besteht ein Bedarf an neuen silanhaltigen Bindemittel, die über spezielle Eigenschaften verfügen.

Aufgabe dieser Erfindung war es, neue silanhaltige Verbindungen zugänglich zu machen, die für die Entwicklung hochvernetzter, harter Beschichtungen geeignet sind.

Diese Aufgabe wird durch Alkoxysilan-funktionalisierte Allophanate gemäß der vorliegenden Erfindung gelöst.

Überraschenderweise wurde gefunden, dass das erfindungsgemäße Alkoxysilan-funktionalisierte Allophanat für die Anwendung als Lack-, Klebstoff- oder Dichtstoff geeignet ist. Insbesondere kann das erfindungsgemäße Alkoxysilan-funktionalisierte Allophanat für die Entwicklung hochvernetzter, besonders harter Beschichtungen eingesetzt werden. Hierbei kann das erfindungsgemäße Alkoxysilan-funktionalisierte Allophanat als alleiniges Bindemittel sowohl in der Kalt- als auch Heißhärtung, bei Bedarf sogar lösemittelfrei, eingesetzt werden.

Gegenstand der Erfindung sind Alkoxysilan-funktionalisierten Allophanate enthaltend das Reaktionsprodukt aus
A) Alkoxysilangruppen haltige Monourethane A) der Formel 1

   Rₙ (OR¹)₃₋ₙ Si-R²-NH-(C=O)-OR³ Formel 1

   wobei Rₙ, R¹, R² und R³ unabhängig voneinander Kohlenwasserstoffreste mit 1-8 C-Atomen bedeuten, wobei diese linear, verzweigt oder cyclisch sein können, oder auch cyclisch ineinander übergehen können, und n gleich 0-2 bedeutet,
   und
B) mindestens einem Diisocyanat B),
in einem Mol-Verhältnis von A) zu B) von 3:1 bis 1,5 :1, bevorzugt 2,5:1 bis 1,8 :1, besonders bevorzugt 2:1.

Bevorzugter Gegenstand der Erfindung sind Alkoxysilan-funktionalisierten Allophanate bestehend aus dem Reaktionsproduktes aus A) und B) wie oben definiert, in einem Mol-Verhältnis von A) zu B) von von 3:1 bis 1,5 :1, bevorzugt 2,5 :1 bis 1,8 :1, besonders bevorzugt 2 :1.

Gegenstand der Erfindung sind auch Alkoxysilan-funktionalisierte Allophanate, erhalten durch Umsetzung von
A) Alkoxysilangruppen haltige Monourethane A) der Formel 1

   Rₙ (OR¹)₃₋ₙ Si-R²-NH-(C=O)-OR³ Formel 1

   wobei Rₙ, R¹, R² und R³ unabhängig voneinander Kohlenwasserstoffreste mit 1-8 C-Atomen bedeuten, wobei diese linear, verzweigt oder cyclisch sein können, oder auch cyclisch ineinander übergehen können, und n gleich 0-2 bedeutet,
   und
B) mindestens einem Diisocyanat B),
   in einem Mol-Verhältnis von A) zu B) von 3:1 bis 1,5 :1, bevorzugt 2,5 :1 bis 1,8 :1, besonders bevorzugt 2:1;
C) optional in Gegenwart von mindestens eines Katalysators C),
   und
D) optionale Umsetzung der Restmenge an NCO-Gruppen aus B) mit einem Alkohol D).

Bevorzugt sind Rₙ, R¹, R² und R³ gleichzeitig oder unabhängig voneinander Methyl, Ethyl, n-Propyl, iso-Propyl, n-Butyl, sec-Butyl, oder tert-Butyl.
Bevorzugt ist n = 0.
R¹ und R³ sind bevorzugt gleichzeitig oder unabhängig voneinander Methyl oder Ethyl.
R² ist bevorzugt Methyl oder Propyl.
Bevorzugt sind Verbindungen mit n gleich 0, R¹ und R³ gleichzeitig oder unabhängig voneinander gleich Methyl oder Ethyl, und R² gleichzeitig oder unabhängig voneinander gleich Methyl oder Propyl.
Bevorzugt ist R³ = R¹.
Bevorzugt sind Verbindungen mit n gleich 0 und R² gleich Methyl oder Propyl, und R¹ gleich Methyl oder Ethyl und R³ = R¹.

Ganz besonders bevorzugt ist die Verbindung n gleich 0, R¹ und R³ gleich Methyl und R² gleich Propyl, N-Trimethoxysilylpropylmethylcarbamat.

Nach erfindungsgemäßer Umsetzung des Alkoxysilangruppen haltigen Monourethans A) mit dem Diisocyanat B) beträgt der NCO-Gehalt im Endprodukt <3 Gew.-%, besonders bevorzugt <1 Gew.-%, ganz besonders bevorzugt <0,2 Gew.-%.

Das erfindungsgemäß eingesetzte Diisocyanat B) kann ein beliebiges aliphatisches, cycloaliphatisches und/oder (cyclo)aliphatisches Diisocyanat sein. In einer bevorzugten Ausführungsform wird unter dem Begriff "(cyclo)aliphatisches Diisocyanat", wie hierin verwendet, verstanden, dass in einem Molekül gleichzeitig an einen Ring gebundene NCO-Gruppen und an einen aliphatischen Rest gebundene NCO-Gruppen vorhanden sind, wie es z. B. beim Isophorondiisocyanat der Fall ist. In einer bevorzugten Ausführungsform wird unter dem Begriff "cycloaliphatisches Diisocyanat", wie hierin verwendet ein Diisocyanat verstanden, das nur direkt am cycloaliphatischen Ring gebundene NCO-Gruppen aufweist, z. B. Diisocyanatodicyclohexylmethan (H12MDI).

Zur Verwendung als Diisocyanat B) geeignete aliphatische Diisocyanate umfassen lineare und/oder verzweigte Alkylenrest mit bevorzugt 3 bis 16 Kohlenstoffatome, bevorzugter 4 bis 12 Kohlenstoffatome. Geeignete cycloaliphatische oder (cyclo)aliphatische Diisocyanate umfassen einen Cycloalkylenrest mit bevorzugt 4 bis 18 Kohlenstoffatomen, bevorzugter 6 bis 15 Kohlenstoffatomen. Beispiele geeigneter Disocyanate umfassen Cyclohexandiisocyanat, Methylcyclohexandiisocyanat, Ethylcyclo-hexandiisocyanat, Propylcyclohexandiisocyanat, Methyldiethylcyclohexandiisocyanat, Propandiisocyanat, Butandiisocyanat, Pentandiisocyanat, Hexandiisocyanat, Heptandiisocyanat, Octandiisocyanat, Nonandiisocyanat, wie 4-Isocyanatomethyl-1,8-octandiisocyanat (TIN), Dekandi- und triisocyanat, Undekandi- und-triisocyanat, Dodecandi- und -triisocyanate. Ebenfalls geeignet sind 4-Methyl-cyclohexan-1,3-diisocyanat, 2-Butyl-2-ethylpentamethylen-diisocyanat, 3(4)-Isocyanatomethyl-1-methylcyclohexylisocyanat, 2-Isocyanatopropylcyclohexyl-isocyanat, 2,4'-Methylenbis(cyclohexyl)diisocyanat und/oder 1,4-Diisocyanato-4-methyl-pentan.

Bevorzugte Diisocyanate B) sind Isophorondiisocyanat (IPDI), Hexamethylendiisocyanat (HDI), 2,2'-Dicyclohexylmethandiisocyanat (2,2'-H12MDI), 2,4'-Dicyclohexylmethandiisocyanat (2,4'-H12MDI), 4,4'-Dicyclohexylmethandiisocyanat (4,4'-H12MDI), 2-Methylpentandiisocyanat (MPDI), Pentandiisocyanat, 2,2,4-Trimethylhexamethylendiisocyanat (2,2,4-TMDI), 2,4,4-Trimethylhexamethylendiisocyanat (2,4,4-TMDI), Norbornandiisocyanat (NBDI), Methylendiphenyldiisocyanat (MDI), Toluidindiisocyanat (TDI), Tetramethylxylylendiisocyanat (TMXDI), Xylylendiisocyanat (MXDI), einzeln oder Mischungen davon.

In einer besonders bevorzugten Ausführungsform ist das Diisocyanat B) IPDI und/oder 4,4'-H12MDI und/oder HDI und/oder ein Gemisch von 2,2,4-TMDI und 2,4,4-TMDI.

Gegenstand der Erfindung ist auch ein Verfahren zur Herstellung von Alkoxysilan-funktionalisierten Allophanaten durch Umsetzung von
A) Alkoxysilangruppen haltige Monourethane A) der Formel 1

   Rₙ (OR¹)₃₋ₙ Si-R²-NH-(C=O)-OR³ Formel 1

   wobei Rₙ, R¹, R² und R³ unabhängig voneinander Kohlenwasserstoffreste mit 1-8 C-Atomen bedeuten, wobei diese linear, verzweigt oder cyclisch sein können, oder auch cyclisch ineinander übergehen können, und n gleich 0-2 bedeutet,
   und
B) mindestens einem Diisocyanat B),
   in einem Mol-Verhältnis von A) zu B) von 3:1 bis 1,5 :1, bevorzugt 2,5 :1 bis 1,8 :1, besonders bevorzugt 2 :1;
C) optional in Gegenwart von mindestens eines Katalysators C),
   und
D) optionale Umsetzung der Restmenge an NCO-Gruppen aus B) mit einem Alkohol D).

Die Herstellung der erfindungsgemäßen Monoallophanate erfolgt im Allgemeinen lösungsmittelfrei oder unter Verwendung von nicht-protischen Lösemitteln, wobei die Umsetzung diskontinuierlich oder kontinuierlich erfolgen kann. Die Reaktion wird durchgeführt in geeigneten Aggregaten, z.B. Rührkessel, Extruder, Statikmischern, Knetkammern. Die Reaktion kann bei Raumtemperatur, das heißt bei Temperaturen im Bereich von 15 bis 40 °C, insbesondere im Bereich von 15 bis 25 °C, durchgeführt werden. Bevorzugt werden jedoch höhere Temperaturen im Bereich 80 bis 220 °C, insbesondere im Bereich von 80 bis 120 °C verwendet. Die Reaktion wird unter Ausschluss von Wasser durchgeführt. Bevorzugt wird die Reaktion lösemittelfrei durchgeführt.

Zur Beschleunigung der Reaktion können vorteilhaft in der Urethanchemie bekannte Katalysatoren C), z.B. Metallorganische Verbindungen, wie Zinn oder Zink haltige Verbindungen, Salze, wie z.B. Zn(II)chlorid und/oder Basen verwendet werden. Geeignet sind zum Beispiel Sn-, Bi-, Zn- und andere Metallcarboxylate wie z.B. Dibutylzinndilaurat, Zinnoctoat, Zinkethylhexanoat, Bismuthneodecanoat, tert.-Amine wie beispielsweise 1,4-Diazabicyclo[2.2.2]octan (DABCO), 1,8-Diazabicyclo[5.4.0]undec-7-en (DBU), 1,5-Diazabicyclo[4.3.0]non-5-en (DBN), Triethylamin, Amidine und Guanidine, sowie quarternäre Ammoniumsalze, bevorzugt Tetralkylammoniumsalze und/oder quarternäre Phosphoniumsalze .

Als Katalysatoren C) kommen auch Metallacetylacetonate in Frage. Beispiele dafür sind Zinkacetylacetonat, Lithiumacetylacetonat Eisenacetylacetonat und Zinnacetylacetonat, allein oder in Mischungen. Bevorzugt wird Zinkacetylacetonat oder Zinkethylhexanoat eingesetzt. Als Katalysatoren kommen außerdem quaternäre Ammoniumacetylacetonate oder quarternäre Phosphoniumacetylacetonate in Frage.

Nach erfindungsgemäßer Umsetzung des Alkoxysilangruppen haltigen Monourethans A) mit dem Diisocyanat B) beträgt der NCO-Gehalt <3 Gew.-%, besonders bevorzugt <1 Gew.-%, ganz besonders bevorzugt <0,2 Gew.-%. Für den Fall, dass der NCO-Gehalt zwischen 3 Gew.-% und 0,2 Gew.-% liegt, werden die Restmengen an NCO-Gruppen aus B) mit einem Alkohol D) im Verhältnis NCO-Gruppen zu OH-Gruppen des Alkohols D) von 0,8:1 bis 1,2:1, bevorzugt von 0,9:1 bis 1,1:1 umgesetzt, wobei die stöchiometrische Umsetzung, d.h. im Verhältnis 1:1 besonders bevorzugt ist. Bevorzugt erfolgt die Umsetzung der Restmenge an NCO-Gruppen aus B) mit einem Alkohol D) bei Temperaturen im Bereich 30 - 150 °C, insbesondere im Bereich von 50 - 150 °C.

Die Reaktion wird unter Ausschluss von Wasser durchgeführt. Bevorzugt wird die Reaktion lösemittelfrei durchgeführt.

Bevorzugte Alkohole D) sind lineare oder verzweigte Alkohole, deren Hydroxyfunktion an einem primären, sekundären oder tertiären Kohlenstoffatom gebunden ist, eingesetzt. Auch Diole oder Polyole können eingesetzt werden. Besonders bevorzugt sind Methanol, Ethanol, Propanol, Isopropanol, 1-Butanol, 2-Butanol, Pentanol, Ethyl-2-hexanol, 1-Hexanol. Ganz besonders bevorzugt sind Ethanol, Propanol und 1-Butanol.

Gegenstand der Erfindung ist auch Beschichtungsmittel und Klebstoffe, enthaltend oder bestehend aus:
Alkoxysilan-funktionalisierten Allophanate enthaltend das Reaktionsproduktes aus
   A) Alkoxysilangruppen haltige Monourethane A) der Formel 1

      Rₙ (OR¹)₃₋ₙ Si-R²-NH-(C=O)-OR³ Formel 1

      wobei Rₙ, R¹, R² und R³ unabhängig voneinander Kohlenwasserstoffreste mit 1-8 C-Atomen bedeuten, wobei diese linear, verzweigt oder cyclisch sein können, oder auch cyclisch ineinander übergehen können, und n gleich 0-2 bedeutet,
      und
   B) mindestens einem Diisocyanat B),
   in einem Mol-Verhältnis von A) zu B) von 3:1 bis 1,5 :1.

Gegenstand der Erfindung ist auch die Verwendung der erfindungsgemäßen Alkoxysilan-funktionalisierte Allophanate in Beschichtungszusammensetzungen und Lackzusammensetzungen für Metall-, Kunststoff, Glas-, Holz-, MDF- (Middle Density Fiber Boards) oder Ledersubstrate oder sonstigen hitzeresistenten Untergründen.

Gegenstand der Erfindung ist auch die Verwendung der Alkoxysilan-funktionalisierte Allophanate gemäß den Ansprüchen 1- 17 in Klebstoffzusammensetzungen für Verklebungen von Metall-, Kunststoff-, Glas-, Holz-, MDF- oder Ledersubstraten oder sonstigen hitzeresistenten Untergründen.

Die vorliegende Erfindung wird weiterhin durch die folgenden nicht beschränkenden Beispiele veranschaulicht, denen weitere Merkmale, Ausführungsformen, Aspekte und Vorteile der vorliegenden Erfindung entnommen werden können.

### Beispiele:

### Einsatzstoffe:

**Vestanat® EP-UPMS:** Trimethoxysilylpropylmethylcarbamat (Evonik Resource Efficiency GmbH)
**Vestanat® IPDI:** Isophorondiisocyanat (Evonik Resource Efficiency GmbH)
**Vestanat® TMDI:** Mischung aus 2,2,4-Trimethylhexamethylendiisocyanat (2,2,4-TMDI) und 2,4,4-Trimethylhexamethylendiisocyanat (Evonik Resource Efficiency GmbH)
**Vestanat® HT 2500/100:** Hexamethylen-1,6-diisocyanat, homopolymer (Isocyanurat-Typ) (Evonik Resource Efficiency GmbH)
**Vestanat® EP Cat 11 B:** Tetraethylammoniumbenzoat in Butanol (Evonik Resource Efficiency GmbH)
**Tegoglide® 410:** Gleit und Antiblocking Aditiv auf Basis eines Polyethersiloxan Copolymers (Evonik Resource Efficiency GmbH)

### 1. Herstellung

### Beispiel 1

### Alkoxysilan-funktionalisiertes Allophanat 1

Es wurden 340,2 g Vestanat® EP-UPMS, 0,3 g Zink(II)ethylhexanoat und 159,7 g Vestanat® IPDI in einem Dreihalskolben mit Rückflusskühler vorgelegt, mit Stickstoff abgedeckt und unter Rühren auf 100 °C erhitzt. Nach 20-stündigem Erhitzen wurde ein NCO-Gehalt von 1,4 Gew.% NCO erhalten. Dann wurden 10,84 g Butanol hinzugefügt und 1h bei 100 °C erhitzt bis ein NCO-Gehalt von < 0,1 Gew.% NCO erreicht wurde. Nach Abkühlen auf Raumtemperatur erhält man das erfindungsgemäße Alkoxysilan-funktionalisierte Allophanat 1 als klare Flüssigkeit mit einer Viskosität von 14,3 Pas (bei 23°C).

### Beispiel 2

### Alkoxysilan-funktionalisiertes Allophanat 2

Es wurden 474,6 g Vestanat® EP-UPMS, 0,22 g Zink(II)ethylhexanoat und 211,8 g Vestanat® TMDI in einem Dreihalskolben mit Rückflusskühler vorgelegt, mit Stickstoff abgedeckt und unter Rühren auf 100 °C erhitzt. Nach 24-stündigem Erhitzen wurde ein NCO-Gehalt von 0,8 Gew.% NCO erhalten. Dann wurden 10,35 g Butanol hinzugefügt und 3h bei 65 °C erhitzt bis ein NCO-Gehalt von < 0,1 Gew. % NCO erreicht wurde. Nach Abkühlen auf Raumtemperatur erhält man das erfindungsgemäße Alkoxysilan-funktionalisierte Allophanat 2 als klare Flüssigkeit mit einer Viskosität von 1170 mPas (bei 23°C).

### Vergleichsbeispiel 3A

### Alkoxysilan-funktionalisiertes Allophanat 3A (Vergleichsbeispiel)

Es wurden 44,3 g Vestanat® EP-UPMS, 0,01 g Zink(II)ethylhexanoat und 35,7 g Vestanat® HT 2500/100 in einem Dreihalskolben mit Rückflusskühler vorgelegt, mit Stickstoff abgedeckt und unter Rühren auf 100 °C erhitzt bis der NCO-Gehalt von <0,1 Gew.% erreicht wurde. Dann wurde noch in der Hitze zwecks Viskositätserniedrigung 20 g Butylacetat hinzugefügt. Das so erhaltene Alkoxysilan-funktionalisierte Allophanat 3 ist eine klare Flüssigkeit mit einer Viskosität von 750 mPas (bei 23°C).

### 2. Herstellung von Klarlacken aus den Alkoxysilan-funktionalisierten Allophanaten als Beschichtungsmittel

Für die Formulierung der erfindungsgemäßen Klarlacke und der Vergleichsbeispiele wurden die Komponenten der in Tabelle 1 und 2 dargestellten Zusammensetzungen unmittelbar vor der Verarbeitung miteinander gemischt.

Die Viskosität der Formulierungen betrug, bestimmt als Auslaufzeit im DIN-4-Becher bei 23 °C, ca. 60 Sekunden.

**Tabelle 1: Zusammensetzung der erfindungsgemäßen Klarlacken und Vergleichsbeispiel von Raumtemperatur (RT)-härtenden Systemen, Angaben in Gew.-%**

| Position | | I | II | III (Vergleich) |
|---|---|---|---|---|
| 1 | Allophanat 1 | 91,24 | | |
| 2 | Allophanat 2 | | 99,00 | |
| 3 | Vergleichsbeispiel: Allophanat 3 (Vergleich) | | | 98,5 |
| 4 | 1,8-Diazabicyclo[5.4.0]undec-7-en (DBU) | 0,92 | 1,0 | 1,0 |
| 5 | Xylol | 7,79 | | 0,45 |
| 6 | Tegoglide 410 | 0,05 | | 0,45 |

| | | | | |
|---|---|---|---|---|
| Bezogen auf Harz beträgt der Gehalt an Katalysator DBU in den Beispiel I und II 1,0% und in Beispiel III 1,25 % DBU. | | | | |

**Tabelle 2: Zusammensetzung der erfindungsgemäßen Klarlacken und Vergleichsbeispiel von Heiß-härtenden Systemen, Angaben in Gew.-%**

| Position | | IV | V (Vergleich) |
|---|---|---|---|
| 1 | Allophanat 1 | 84,00 | |
| 2 | Allophanat 2 | | |
| 3 | Vergleichsbeispiel: Allophanat 3 (Vergleich) | | 88,53 |
| 4 | Vestanat Cat 11 B | 1,71 | 1,45 |
| 5 | Xylol | 14,29 | 10,02 |

| | | | |
|---|---|---|---|
| Bezogen auf Harz beträgt der Gehalt an Katalysator Vestanat Cat 11 B in allen Beispielen 1,0%. | | | |

Zur Ermittlung der mechanischen Kenndaten wurden alle Lacke mit einer 100 µm Rakel auf phosphatierte Stahlbleche (Chemetall Gardobond 26S/60/OC) appliziert und bei Raumtemperatur (23°C), Tabelle 3, bzw. bei 140°C, Tabelle 4, gehärtet.

**Tabelle 3: Lackeigenschaften der Zusammensetzungen I-III nach Härtung bei 23°C (7 Tage)**

| Zusammensetzung | I | II | III (Vergleich) |
|---|---|---|---|
| Pendelhärte (König) [s] n 7 d | 189 | 168 | 158 |
| MEK-Test [ASTM D 4752] (Doppelhübe, 1 kg Auflagegewicht) | >150 | >150 | >150 |
| Aussehen der Beschichtung | glänzend | glänzend | glänzend |

Die Lackeigenschaften der Beschichtungen I und II, die die erfindungsgemäßen Alkoxysilanfunktionalisiertes Allophanat 1 oder 2 enthalten, zeigen signifikant höhere Pendelhärten als das Vergleichsbeispiel III. Insbesondere bei dem dreifach funktionalisierten Produkt III hätte man aufgrund des höheren Vernetzungsgrades eine höhere Härte erwartet.

**Tabelle 4: Lackeigenschaften der Zusammensetzungen IV - V nach Härtung bei 140 °C (22 min)**

| Zusammensetzung | IV | V(Vergleich) |
|---|---|---|
| Pendelhärte (König) [s] n 1 d | 178 | 118 |
| MEK-Test [ASTM D 4752] (Doppelhübe, 1 kg Auflagegewicht) | >150 | >150 |
| Aussehen der Beschichtung | glänzend | matt |

Die Lackeigenschaften der Beschichtung IV, die das erfindungsgemäße Alkoxysilan-funktionalisierte Allophanat 1 enthält, zeigt eine signifikant höhere Pendelhärte als das Vergleichsbeispiel V. Insbesondere bei dem dreifach funktionalisierten Produkt V hätte man aufgrund des höheren Vernetzungsgrades eine höhere Härte erwartet. Zudem zeigt die Beschichtung IV mit ihrer glänzenden Oberfläche ein besseres Aussehen als die matte Beschichtung V.

Die Ergebnisse aus Tabelle 3 und 4 belegen, dass die erfindungsgemäßen Alkoxysilan-funktionalisierten Allophanate für die Entwicklung hochvernetzter, besonders harter Beschichtungen eingesetzt werden können und nur diese dabei als alleiniges Bindemittel sowohl in der Kalt- als auch Heißhärtung, bei Bedarf sogar lösemittelfrei, eingesetzt werden können.

## Patentansprüche

1. Alkoxysilan-funktionalisierten Allophanate enthaltend das Reaktionsproduktes aus
A) Alkoxysilangruppen haltige Monourethane A) der Formel 1
Rₙ (OR¹)₃₋ₙ Si-R²-NH-(C=O)-OR³ Formel 1
wobei Rₙ, R¹, R² und R³ unabhängig voneinander Kohlenwasserstoffreste mit 1-8 C-Atomen bedeuten, wobei diese linear, verzweigt oder cyclisch sein können, oder auch cyclisch ineinander übergehen können, und n gleich 0-2 bedeutet,
und
B) mindestens einem Diisocyanat B),
in einem Mol-Verhältnis von A) zu B) von von 3:1 bis 1,5 :1.

2. Alkoxysilan-funktionalisierte Allophanate, erhalten durch Umsetzung von
A) Alkoxysilangruppen haltige Monourethane A) der Formel 1
Rₙ (OR¹)₃₋ₙ Si-R²-NH-(C=O)-OR³ Formel 1
wobei Rₙ, R¹, R² und R³ unabhängig voneinander Kohlenwasserstoffreste mit 1-8 C-Atomen bedeuten, wobei diese linear, verzweigt oder cyclisch sein können, oder auch cyclisch ineinander übergehen können, und n gleich 0-2 bedeutet,
und
B) mindestens einem Diisocyanat B),
in einem Mol-Verhältnis von A) zu B) von 3:1 bis 1,5 :1, bevorzugt 2,5 :1 bis 1,8 :1, besonders bevorzugt 2:1;
C) optional in Gegenwart von mindestens eines Katalysators C),
und
D) optionale Umsetzung der Restmenge an NCO-Gruppen aus B) mit einem Alkohol D).

3. Alkoxysilan-funktionalisierte Allophanate nach Anspruch 1 oder 2, wobei das Mol-Verhältnis von A) zu B) von 2,5 :1 bis 1,8 :1, besonders bevorzugt von 2 :1, beträgt.

4. Alkoxysilan-funktionalisierte Allophanate nach mindestens einem der vorherigen Ansprüche , wobei Rₙ, R¹, R² und R³ gleichzeitig oder unabhängig voneinander Methyl, Ethyl, n-Propyl, iso-Propyl, n-Butyl, sec-Butyl, oder tert-Butyl, bedeuten.

5. Alkoxysilan-funktionalisierte Allophanate nach mindestens einem der vorherigen Ansprüche, wobei n gleich 0, R¹ und R³ gleichzeitig oder unabhängig voneinander gleich Methyl oder Ethyl, und R² gleichzeitig oder unabhängig voneinander gleich Methyl oder Propyl, bedeuten.

6. Alkoxysilan-funktionalisierte Allophanate nach mindestens einem der vorherigen Ansprüche, wobei gleich 0 und R² gleich Methyl oder Propyl, und R¹ gleich Methyl oder Ethyl und R³ = R¹ bedeuten.

7. Alkoxysilan-funktionalisierte Allophanate nach mindestens einem der vorherigen Ansprüche, wobei n gleich 0, R¹ und R³ gleich Methyl und R² gleich Propyl bedeuten.

8. Alkoxysilan-funktionalisierte Allophanate nach mindestens einem der vorherigen Ansprüche, das Diisocyanate B) ausgewählt aus Isophorondiisocyanat (IPDI), Hexamethylendiisocyanat (HDI), 2,2' Di-cyclohexylmethandiisocyanat (2,2'-H12MDI), 2,4' Dicyclohexylmethandiisocyanat (2,4'-H12MDI), 4,4' Dicyclohexylmethandiisocyanat (4,4'-H12MDI), 2 Methylpentandiisocyanat (MPDI), Pentandiisocyanat, 2,2,4-Trimethylhexamethylendiisocyanat (2,2,4-TMDI), 2,4,4-Trimethylhexamethylendiisocyanat (2,4,4 TMDI), Norbornandiisocyanat (NBDI), Methylendiphenyldiisocyanat (MDI), Toluidindiisocyanat (TDI), Tetramethylxylylendiisocyanat (TMXDI), Xylylendiisocyanat (MXDI), einzeln oder Mischungen davon, eingesetzt werden.

9. Alkoxysilan-funktionalisierte Allophanate nach mindestens einem der vorherigen Ansprüche, das Diisocyanat B) ausgewählt aus IPDI, 4,4'-H12MDI, HDI oder ein Gemisch von 2,2,4-TMDI und 2,4,4 TMDI, einzeln oder Mischungen davon, eingesetzt werden.

10. Alkoxysilan-funktionalisierte Allophanate nach mindestens einem der vorherigen Ansprüche, **dadurch gekennzeichnet, dass** als Komponente C) Metallcarboxylate, tert.-Amine, Amidine Guanidine, quarternäre Ammoniumsalze, Tetralkylammoniumsalze, quarternäre Phosphoniumsalze, Metallacetylacetonate, quarternäre Ammoniumacetylacetonate, quarternäre Phosphoniumacetylacetonate, allein oder in Mischungen, eingesetzt werden.

11. Alkoxysilan-funktionalisierte Allophanate nach mindestens einem der vorherigen Ansprüche, **dadurch gekennzeichnet, dass** als Komponente C) Zinkacetylacetonat und/oder Zinkethylhexanoat eingesetzt wird.

12. Alkoxysilan-funktionalisierte Allophanate nach mindestens einem der vorherigen Ansprüche, **dadurch gekennzeichnet, dass** Alkohole D) ausgewählt aus Methanol, Ethanol, Propanol, Isopropanol, 1-Butanol, 2-Butanol, Pentanol, Ethyl-2-hexanol, 1-Hexanol, eingesetzt werden.

13. Verfahren zur Herstellung von Alkoxysilan-funktionalisierten Allophanaten nach mindestens einem der vorherigen Ansprüche durch Umsetzung von
A) Alkoxysilangruppen haltige Monourethane A) der Formel 1
Rₙ (OR¹)₃₋ₙ Si-R²-NH-(C=O)-OR³ Formel 1
wobei Rₙ, R¹, R² und R³ unabhängig voneinander Kohlenwasserstoffreste mit 1-8 C-Atomen bedeuten, wobei diese linear, verzweigt oder cyclisch sein können, oder auch cyclisch ineinander übergehen können, und n gleich 0-2 bedeutet,
und
B) mindestens einem Diisocyanat B),
in einem Mol-Verhältnis von A) zu B) von 3:1 bis 1,5 :1, bevorzugt 2,5 :1 bis 1,8 :1, besonders bevorzugt 2:1;
C) optional in Gegenwart von mindestens eines Katalysators C),
und
D) optionale Umsetzung der Restmenge an NCO-Gruppen aus B) mit einem Alkohol D).

14. Verfahren nach Anspruch 13, wobei die Reaktion bei Temperaturen im Bereich von 15 bis 40 °C, insbesondere im Bereich von 15 bis 25 °C, durchgeführt wird.

15. Verfahren nach Anspruch 13, wobei die Reaktion bei Temperaturen im Bereich 80 bis 220 °C, insbesondere im Bereich von 80 bis 120 °C durchgeführt wird.

16. Verfahren nach Anspruch 13-15, wobei die Umsetzung der Restmenge an NCO-Gruppen aus B) mit einem Alkohol D) bei Temperaturen im Bereich 30 - 150 °C, insbesondere im Bereich von 50 - 150 °C, durchgeführt wird.

17. Verfahren nach Anspruch 13-16, wobei, die Umsetzung in Gegenwart von Zinkacetylacetonat und/oder Zinkethylhexanoat als Katalysator C) durchgeführt wird.

18. Verfahren nach Anspruch 13-17, wobei die Restmengen an NCO-Gruppen aus B) mit einem Alkohol D) im Verhältnis NCO-Gruppen zu OH-Gruppen des Alkohols D) von 0,8:1 bis 1,2:1, bevorzugt von 0,9:1 bis 1,1:1 umgesetzt wird, besonders bevorzugt durch stöchiometrische Umsetzung im Verhältnis von 1:1.

19. Verwendung der Alkoxysilan-funktionalisierte Allophanate gemäß den Ansprüchen 1- 18 in Beschichtungszusammensetzungen und Lackzusammensetzungen für Metall-, Kunststoff-, Glas-, Holz-, MDF- (Middle Density Fiber Boards) oder Ledersubstrate oder sonstigen hitzeresistenten Untergründen.

20. Verwendung der Alkoxysilan-funktionalisierte Allophanate gemäß den Ansprüchen 1- 18 in Klebstoffzusammensetzungen für Verklebungen von Metall-, Kunststoff-, Glas-, Holz-, MDF- oder Ledersubstraten oder sonstigen hitzeresistenten Untergründen.

21. Beschichtungsmittel und Klebstoffe und Dichtstoffe, enthaltend:
Alkoxysilan-funktionalisierten Allophanate enthaltend das Reaktionsproduktes aus
A) Alkoxysilangruppen haltige Monourethane A) der Formel 1
Rₙ (OR¹)₃₋ₙ Si-R²-NH-(C=O)-OR³ Formel 1
wobei Rₙ, R¹, R² und R³ unabhängig voneinander Kohlenwasserstoffreste mit 1-8 C-Atomen bedeuten, wobei diese linear, verzweigt oder cyclisch sein können, oder auch cyclisch ineinander übergehen können, und n gleich 0-2 bedeutet,
und
B) mindestens einem Diisocyanat B),
in einem Mol-Verhältnis von A) zu B) von 3:1 bis 1,5 :1.
